# EUROPÄISCHE PATENTANMELDUNG

(11) **EP 0 902 088 A2**
(43) Veröffentlichungstag der Anmeldung: **17.03.1999**
(21) Anmeldenummer: 98118811.3
(22) Anmeldetag: 26.03.1997
(51) Int. Cl.: C12N 15/62, C12N 15/12, C07K 14/47, C07K 14/475, C07K 14/71, C12Q 1/68, A61K 38/18, A61K 31/70, G01N 33/68

(54) **Petide mit antiproliferativen Eigenschaften**

(30) Priorität: 26.03.1996 DE 19611939; 20.12.1996 DE 19653445
(62) Teilanmeldung aus: 97918058.5
(71) Anmelder: Radulescu, Razvan T., 81375 München (DE)
(72) Erfinder: Radulescu, Razvan T., 81375 München (DE)
(74) Vertreter: Straus, Alexander, Dr. Dipl.-Chem.

(57) **Zusammenfassung**

Die vorliegende Erfindung betrifft antiproliferative Peptide, die sich von einem Tumorsuppressorprotein ableiten und an Wachstumsfaktor- oder Wachstumsfaktor-Rezeptor-Segmente binden. Weiter betrifft die Erfindung Nukleinsäuren (DNAs/RNAs), die für diese Peptide kodieren sowie dazu strukturhomologe Peptidnukleinsäuren und pharmazeutische Zusammensetzungen, die die Peptide enthalten. Die Erfindung findet Verwendung in der Biotechnologie, der Molekularbiologie, der Bioinformatik und der Diagnose und Therapie von hyperproliferativen Erkrankungen, insbesondere von Krebs und der Atherosklerose.

## Beschreibung

Die vorliegende Erfindung betrifft Peptide mit antiproliferativen Eigenschaften, die sie kodierenden Nukleinsäure (DNAs/RNAs), zu diesen Nukleinsäuren strukturhomologe Peptidnukleinsäuren sowie die Verwendung der Peptide, der Nukleinsäuren, der Peptidnukleinsäuren und/oder ihrer pharmazeutischen Zusammensetzung in der Biotechnologie, der Molekularbiologie, der Bioinformatik und der Diagnose und Therapie von hyperproliferativen Erkrankungen, insbesondere von Krebs und der Atherosklerose.

Im Bereich der Onkologie gibt es derzeit zahlreiche feststehende Behandlungsschemata, die vor allem auf Operationsverfahren, Chemo- und Strahlentherapie basieren. Allerdings haben diese Methoden noch nicht den gewünschten Durchbruch in der Krebstherapie gebracht. Laut einer aktuellen amerikanischen Statistik erkrankt jeder dritte lebende Amerikaner an Krebs und jeder fünfte stirbt daran. Diese Zahl dürfte für die Industrienationen weltweit zutreffen und ist schon seit mehr als einem Jahrzehnt relativ unverändert. Weiterhin ist festzuhalten, daß sich seit zwanzig Jahren die Effizienz der Krebsbehandlung nicht deutlich verbessert hat, da die Fünf-Jahres-Überlebensraten für die Mehrzahl der Krebsarten ungefähr gleich geblieben sind (R.N. Proctor "The Sciences", März/April 1995, pp. 20-24).

Die Aufgabe der vorliegenden Erfindung besteht deshalb darin, eine Möglichkeit bereitzustellen, mit der das Wachstum vieler verschiedener Krebsarten effizient gebremst werden kann und es zu einer Rückbildung der Tumore kommt.

Gelöst wird diese Aufgabe durch ein Peptid gemäß Anspruch 1, durch eine Nukleinsäure (DNA/RNA) gemäß Anspruch 9, durch eine Peptidnukleinsäure gemäß Anspruch 12, durch eine pharmazeutische Zusammensetzung gemäß Anspruch 13 sowie durch die Verwendung der Peptide gemäß Anspruch 15, die Verwendung der DNA/RNA gemäß Anspruch 17, die Verwendung der Peptidnukleinsäure gemäß Anspruch 18 und der pharmazeutischen Zusammensetzung gemäß Anspruch 21. Vorteilhafte Ausgestaltungen ergeben sich aus den Unteransprüchen.

Von dem Erfinder wurde gefunden, daß ein vielversprechender Ansatz in der Behandlung von Krebs darin besteht, spezifische Peptide einzusetzen, die krebsverursachende Substanzen, insbesondere onkogene Proteine blockieren. Die erfindungsgemäßen Peptide zeichnen sich dadurch aus, daß sie eine geringe Sequenzlänge haben und damit vom Synthesestandpunkt her gesehen ökonomisch sind. Außerdem können sie effizient intrazellulär penetrieren, um damit die innerhalb der Zelle lokalisierten, den Krebsprozeß unmittelbar ankurbelnden Zielproteine zu neutralisieren.

Hinter der Erfindung steht die Erkenntnis, daß bei vielen Krebs arten Tumorsuppressorgene deletiert oder mutiert sind, so daß deren Genprodukte nicht in ausreichender Menge vorhanden sind, was zur Krebsentwicklung führt (A.G. Knudson, Proc. Natl. Acad. Sci., USA, 1993, Vol. 90, pp. 10914-10921; A.J Levine, Sci. Am. "Science & Medicine", Jan./Febr. 1995, pp. 28-37). Bei anderen hyperproliferativen Erkrankungen wie z.B. der Atherosklerose scheint ebenfalls ein Defekt von Tumorsuppressorproteinen, z.B. von RB1 pathogenetisch von Bedeutung zu sein (Chang, M.W. et al. Science 1995 Vol. 267, pp. 518-522). Vom Erfinder wurde nun gefunden, daß es genügt, Teile eines Tumorsuppressorproteins in die Zelle einzuführen, um Wachstumsfaktoren, die das unkontrollierte Zellwachstum beschleunigen, zu hemmen. Man kann nämlich nicht das gesamte Tumorsuppressorprotein in die Zelle einbringen, da ein solches Protein zu lang ist und meist einer proteolytischen Spaltung unterliegt, bevor es seine Wirkung entfalten kann. Verwendet man andererseits nur Teile aus dem Tumorsuppressorprotein werden auch diese zu schnell abgebaut, d.h. sie sind nicht ausreichend stabil.

Bei den erfindungsgemäßen Peptiden handelt es sich deshalb um synthetische Fusionspolypeptide aus den Bausteinen (A) und (B), wobei diese Bausteine in Kombination von AB oder BA vorliegen können. Der erste Baustein (A) enthält einen wirksamen Abschnitt eines Tumorsuppressorproteins - so wie vom Erfinder erstmalig herausgefunden - bzw. dazu hydropathisch homolog ist. Der zweite Teil (B) weist eine Sequenz auf, die den ersten Teil (A) stabilisiert. Erfindungsgemäß bindet das erfindungsgemäße Peptid an einen Wachstumsfaktor oder Wachstumsfaktor-Rezeptor, bevorzugt an eine LXCXE-Sequenz darin, vor allem an die LXCXE-Sequenz in Insulin und zwar an LVCGE (Radulescu et al., Biochemical and Biophysical Research Communications 1995, Vol. 206, pp. 97-102). Da die Wachstumsfaktoren IGF-1 und IGF-2 die Sequenz FVCGD beherbergen, die hydropatisch homolog zum Abschnitt LVCGE in Insulin ist (R. Radulescu & C. Wendtner, J. Mol. Recognition 1992, Vol 5, pp. 133-137), bindet das erfindungsgemäße Peptid auch an IGF-1 und IGF-2 an.

Der oben verwendete Begriff "hydropathisch homolog" wird im Sinne der Lehren von Kyte und Doolittle (J. Kyte & R.F. Doolittle, J. Mol. Biol. 1982, Vol. 157, pp. 105-132) und J.E. Blalock und Smith, E.M. (Biochemical and Biophysical Research Communications, Vol. 121, No. 1, S. 203-207 (1984)) verwendet.

Weiter zeichnet sich der Teil (A) des erfindungsgemäßen Peptids dadurch aus, daß er im Sinne der Komplementärpeptid-Theorie (J.E. Blalock, Trends in Biotechnology, Vol. 8, pp. 140-144, Juni 1990) bzw. im Sinne der dreidimensionalen Konfiguration zu einem Fragment eines Wachstumsfaktors oder Wachstumsfaktor-Rezeptors hydropathisch komplementär ist. Beispiele für Wachstumsfaktoren sind: Insulin, IGF1, IGF2, EGF, FGF, Angiogenin, NGF und PDGF.

Erfindungsgemäß können die für Teil (A) des erfindungsgemäßen Peptids in Frage kommenden wirksamen Abschnitte aus den folgenden Tumorsuppressorgen-Produkten stammen: RB1, P107, P130, WT1, TP53, NF1, NF2, VHL, APC, NB1, MLM, MEN1, BCNS, RCC, BRCA1, BRCA2, DCC, MTS1=p16, MTS 2, P21, P27 und anderen. Bevorzugt umfaßt der Teil (A) einen Abschnitt aus RB1 und zwar die Aminosäuren 649-654 von RB1, d.h.: LFYKKV bzw. ist hydropathisch homolog dazu.

Der Teil (B) des Fusionspeptids sorgt als Kofaktor dafür, daß der Teil (A) stabilisiert wird, insbesondere daß es zu keinem proteolytischen Abbau in der Zelle kommt. Dies kann einerseits dadurch geschehen, daß die erfindungsgemäßen Peptide schnell in den Zellkern hineingelangen und dort gegenüber Proteasen weniger anfällig sind (R. Fahraeus et al., Current Biology 1996, Vol. 6, No. 1, pp 84-91)oder indem bei verzweigten Teilen (B) z.B. Polylysin-Verzweigungen bzw.- Core oder D-Aminosäuren verwendet werden. In bevorzugter Weise ist der Teil (B) ein Polylysin-Core (R. Radulescu et al., Biochemical and Biophysical Research Communications, 1995, Vol. 206, pp. 97-102 sowie G. Fassina, EPO 0 481 930 A2) oder eine nukleäre Lokalisationssequenz (NLS), insbesondere eine SV40-NLS oder Penetratin oder eine bipartite NLS oder die RNP A1 NLS (M9 Region). Allgemeine Hinweise für die Konstruktion von Peptiden, die bevorzugt in den Zellkern hineingelangen sollen, ergeben sich aus Sheldon et al., Proc. Natl., Acad. Sci. USA, Vol. 92, S. 2056-2060 (1995); Dingwall et al., Trends in Biochemical Sciences 16, S. 478-481 (1991) M.S. Moore, Current Biology, Vol. 6, No. 2, S. 137-140 (1996); D.A. Jans, FASEB Journal, 1994, Vol. 8, pp. 841-847; J. Moroianu & J.F. Riordan PNAS, 1994, Vol. 91, pp. 1677-1681 und D. Derossi et al., Journal of Biological Chemistry, 1994, Vol. 269, pp. 10444-10450. Bevorzugt hat der Teil (A) folgende Sequenz bzw. ist dazu in mindestens zwei Positionen hydropathisch homolog:

NH₂-L-F-Y-K-K-V-COOH (P1)

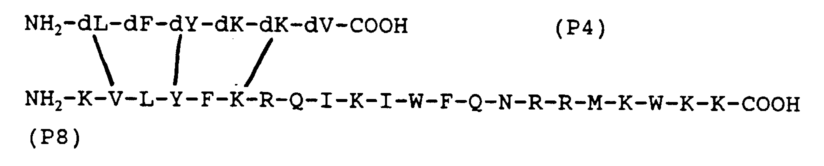

(Striche kennzeichnen eine hydropathische Homologie)

Daraus ergeben sich folgende bevorzugte erfindungsgemäße Peptide:

NH₂-[L-F-Y-K-K-V-GGG]₄-[K-R-G]₂-K-G-COOH (P2)

Dieses Peptid ist eine Kombination aus der Sequenz (P1) und dem Polylysin-Core [-GGG]₄-[K-R-G]₂-K-G.

NH₂-[L-F-Y-K-K-V-GGG]₄-[K]₂-K-G-COOH (P2i)

Dieses Peptid ist eine Kombination aus der Sequenz P1 und dem Polylysin-Core [-GGG]₄-[K]₂-K-G.

NH₂-[dL-dF-dY-dK-dK-dV-GGG]₄- [lK-dR-G]₂-lK-G-COOH (P3)

Dieses Peptid ist eine Kombination aus der Sequenz (P4) und dem Polylysin-Core [-GGG]₄-[1K-dR-G]₂-1K-G.

NH₂- [dL-dF-dY-dK-dK-dV-CGG]₄-lK]₂-lK-G-COOH (P3i)

Dieses Peptid ist eine Kombination aus der Sequenz (P4) und dem Polylysin-Core [-GGG]₄-[1K]₂-1K-G.

NH2-L-F-Y-K-K-V-P-K-K-K-R-K-V-COOH (P5)

Dieses Peptid (P5) setzt sich zusammen aus der Sequenz (P1) und der nukleären Lokalisationssequenz des Large T Antigen des SV40-Virus.

NH₂-L-F-Y-K-K-V-R-Q-I-K-I-W-F-Q-N-R-R-M-K-W-K-K-COOH (P6)

Dieses Peptid (P6) ist eine Kombination aus der Sequenz (P1) und Penetratin, einer 16 Aminosäuren langen Sequenz innerhalb der Antennapedia-Homeodomäne, die der Membrantranslokation und somit auch der nukleären Lokalisation dient (s.o.).

NH₂-[dK-dV-dL-dY-dF-dK-GGG ]₄-[lK-dR-G]₂-lK-G-COOH (P7s)

Dieses Peptid (P7s) ist eine Kombination aus der Sequenz dK-dV-dL-dY-dF-dK, die hydropathisch homolog ist zu P1 und dem Polylysin-Core [-GGG]₄-[lK-dR-G]₂-lK-G.

NH₂-[dK-dV-dL-dY-dF-dK-GGG]₄-[lK]₂-lK-G-COOH (P7)

Dieses Peptid (P7) ist eine Kombination aus der Sequenz dK-dV-dL-dY-dF-dK, die hydropathisch homolog ist zu P1 und dem Polylysin-Core [-GGG]₄-[lK]₂-lK-G.

NH₂-K-V-L-Y-F-K-R-Q-I-K-I-W-F-Q-N-R-R-M-K-W-K-K-COOH (P8)

Dieses Peptid (P8) ist eine Kombination aus der Sequenz dK-dV-dL-dY-dF-dK, die hydropathisch homolog ist zu P1 und aus Penetratin.

"d" und "l" bezeichnen die Konfiguration der im Ein-Buchstaben-Code, dargestellten Aminosäuren.

Allgemein weisen die erfindungsgemäßen Peptide L-und/oder D-Aminosäuren auf. Erfindungsgemäß sollen all-L-Formen, all-D-Formen, die retro-inverso-Isomere sowie die entsprechenden Permutationen von L- und D-Form jeder einzelnen Aminosäure möglich sein. Darüberhinaus sollen erfindungsgemäß alle Permutationen von oxidierter und reduzierter Form jeder einzelnen Aminosäure sowie von freier und Schutzgruppe(n) tragender Aminosäure möglich sein. Die optimalen Wirkkonzentrationen der erfindungsgemäßen Peptide sind erfindungsgemäß im Bereich von 10⁻⁴M bis 10⁻⁵M, andere optimale Wirkkonzentrationsbereiche sind allerdings je nach Anwendungsbereich erfindungsgemäß auch möglich. Für verzweigte Peptide hat es sich als vorteilhaft herausgestellt, daß sie in der all-D-Form sind. Lineare, eine NLS enthaltende Peptide gehen höchstwahrscheinlich in den Zellkern hinein; verzweigte Peptide gehen wahrscheinlich in den Zellkern, können aber auch außerhalb davon ihre Wirkung entfalten.

Die erfindungsgemäßen Peptide kann man als "SCAPs" bezeichnen, d.h. "Synthetische Cofaktor-verbundene antionkogene Peptide".

Die Herstellung der erfindungsgemäßen Peptide erfolgt bevorzugt synthetisch nach der üblichen Solid-Phase-Methode (siehe G.A. Grant, "Synthetic Peptides", W.H. Freeman and Company, New York, 1992). Die anschließende Reinigung der erfindungsgemäßen Peptide wurde durchgführt und überprüft wie schon beschrieben (R. Radulescu et al., Biochemical and Biophysical Research Communications, 1995, Vol. 206, pp. 97-102). Letztere Literaturangabe liefert auch ein Beispiel, wie man die erfindungsgemäßen Peptide zur biotechnologischen Reingewinnung von Wachstumsfaktoren, z.B. von Insulin, und Wachstumsfaktor-Rezeptoren einsetzen kann. Die eine NLS tragenden erfindungsgemäßen Peptide könnte man auch an eine Heparansulfatmatrix tragende Chromatographiesäule koppeln zwecks biotechnologischer Reingewinnung von Wachstumsfaktoren und Wachstumsfaktor-Rezeptoren.

Die Teile (A) und (B) der erfindungsgemäßen Peptide können allerdings auch nach Standardmethoden aus den entsprechenden Proteinen herausgespalten werden und miteinander verbunden werden. Weitere Techniken zur Herstellung der Peptide sind dem Fachmann geläufig (siehe G.A. Grant, "Synthetic Peptides", W.H. Freeman and Company, New York, 1992).

Die Erfindung betrifft weiter eine DNA/RNA kodierend für die erfindungsgemäßen Proteine, wobei die DNA/-RNA-Sequenz über den genetischen Code abgeleitet werden kann.

In bevorzugter Weise kodiert die folgende DNA/RNA für die oben angegebenen Aminosäurensequenzen (P1) und (P4) des Teils (A) eines erfindungsgemäßen Peptids:

5′-CUU UUC UAC AAG AAG GUU-3′ (D1)

In bevorzugter Weise kodiert die folgende DNA/RNA für das oben angegebene erfindungsgemäße Peptid (P5):

5′-CUU UUC UAC AAG AAG GUU CCU AAG AAG AAG CGU AAG
GUU-3′ (D5)

In bevorzugter Weise kodiert die folgende DNA/RNA für das oben angegebene erfindungsgemäße Peptid (P6) :

5′-CUU UUC UAC AAG AAG GUU CGU CAA AUA AAG AUA UGG
UUC CAA AAU CGU CGU AUG AAG UGG AAG AAG-3′ (D6)

In bevorzugter Weise kodiert die folgende DNA/RNA für das oben angegebene erfindungsgemäße Peptid (P8) :

5′-AAG GUU CUU UAC UUC AAG CGU CAA AUA AAG AUA UGG
UUC CAA AAU CGU CGU AUG AAG UGG AAG AAG-3′  (D8)

Die für die erfindungsgemäßen Peptide kodierenden DNAs/RNAs können auch in entsprechende Vektoren eingebaut werden zwecks Einsatz in der Gentherapie gegen Krebs. Eine Übersicht der Methodologie findet sich bei R.C. Mulligan, Science 1993, Vol. 260, pp. 926-932.

Die oben angegebenen DNA/RNA-Sequenzen schließen auch damit unter stringenten Bedingungen, wie sie dem Fachmann geläufig sind, hybridisierende DNAs/RNAs ein. Insbesondere DNAs/RNAs, die bei etwa 20 °C unter dem Schmelzpunkt der DNA/RNA mit den oben angegebenen DNAs/RNAs hybridisieren. Außerdem schließen die oben angegebenen DNA/RNA-Sequenzen DNAs/RNAs ein, die mit den oben angegebenen DNA/RNA-Sequenzen DNAs/RNAs über den degenerierten genetischen Code verwandt sind.

Eine weitere Anwendung der Erfindung ergibt sich für die Bioinformatik und Molekularbiologie. Um Tumorsuppressorproteine zu identifizieren, die mit einem Wachstumsfaktor oder dessen Rezeptor interagieren, könnte man folgende Strategie anwenden. Die cDNA des betreffenden Wachstumsfaktors bzw. dessen Rezeptors könnte man aus der NCBI-Datenbank abrufen und im Sinne der Komplementärpeptidstrategie diese cDNA in eine komplementäre DNA umschreiben und dann in ein Peptid übersetzen mit Hilfe der DNA Strider Software. Für die sich daraus ergebenden Komplementärpeptide könnte man homologe Proteine/Peptide finden (die gesuchten Tumorsuppressoren) mit Hilfe des BLAST-Algorhythmus im OWL-Datenbanknavigator. Umgekehrt könnte man auch vorgehen, in dem man von einer Tumorsuppressor-cDNA ausgeht und analog zum obigen Vorgehen dafür Wachstumsfaktoren bzw. deren Rezeptoren als Interaktionspartner ausfinding macht. Dieses Verfahren könnte auch das Klonieren von (neuen) Tumorsuppressororganen oder (neuen) Wachstumsfaktor- bzw. Wachstumsfaktor-Rezeptor-Genen wesentlich beschleunigen und erleichtern. Beispiel: Die cDNA des humanen EGF-Vorläufer-Proteins wurde aus der NCBI-Datenbank herauskopiert und in die DNA Strider Software hineinkopiert, wo mit Hilfe dieser Software die komplementäre(n) DNA(s) zu der EGF-Vorläufer-cDNA abgeleitet wurden, und diese DNA(s) in Peptide übersetzt wurden, in sogenannte Komplementärpeptide zum EGF-Vorläufer-Protein. In einem weiteren Schritt wurde nach homologen Peptiden/-Proteinen zu diesen Komplementärpeptiden mit Hilfe des BLAST-Algorhythmus im OWL-Datenbanknavigator gesucht. Das Ergebnis dieser Suche war, daß mehrere nukleäre Proteine zum EGF-Vorläufer homolog waren, darunter das mit RB1 strukturell und funktionell verwandte p130-Protein, genauer die p130-Aminosäuren 290-313. Noch genauer: Die EGF-Vorläufer-Aminosäuren 209-213 (REGSN) und die p130-Aminosäuren 305-309 (IGTLS) sind hydropathisch komplementär zueinander und damit potentielle Bindungsstellen in der möglichen Komplexbildung zwischen dem EGF-Vorläufer und p130, wenn man diese Regionen antiparallel zueinander anordnet. Damit könnte die Aminosäuresequenz IGTLS bzw. hydropathisch homologe Sequenzen dazu als Teil (A) eines antiproliferativen Peptids im Sinne der vorliegenden Erfindung dienen (s. Anspruch 1 u. 2).

Für die therapeutische Anwendung werden die erfindungsgemäßen Peptide, einzeln oder in Kombination von mehreren, üblicherweise mit Hilfs-, Füll- und/oder Zusatzstoffen zusammen in einer pharmazeutischen Zusammensetzung verwendet. Besonders vorteilhaft hat sich die Kombination der oben angegebenen Peptide P3 und P6 bzw. P3 und P5 herausgestellt. Als Darreichungsformen der pharmazeutischen Zusammensetzung kommen dabei in Frage: Salben, Lösungen, Dispersionen, Emulsionen, Aerosole, Schäume, teilchenförmige Mittel (z.B. Granulate, Agglomerate, Puder, Mikroperlen, Adsorbate), Pillen, Pastillen, Tabletten, Dragees oder Kapseln. Die erfindungsgemäßen Peptide können auch in Kombination mit anderen Zytostatika oder in Kombination mit Bestrahlungsmethoden verwendet werden.

Die Verabreichung erfolgt vorzugsweise lokal, intracutan oder transcutan; zur systemischen Anwendung vorzugsweise intravenös, intraarteriell, oral, rectal; zur Anwendung in Hohlräumen vorzugsweise intrathekal, intraperitoneal oder intracavitär.

Die erfindungsgemäßen Peptide, DNAs/RNAs und pharmazeutische Zusammensetzung eignen sich als Krebstherapeutika und werden erfindungsgemäß in dieser Weise verwendet, wobei sich ein ausgeprägter zytotoxischer Effekt zeigt.

Bevorzugte Wirksamkeit besteht gegen Mammakarzinom-, Osteosarkom- und Leukämiezellen. Aufgrund seiner Konzeption wirkt das erfindungsgemäße Peptid allgemein gegen alle Tumorzellen, die ein defektes Retinoblastomgen bzw. -protein aufweisen.

Die Erfindung wird weiter anhand der 15 Figuren und 2 Tabellen beschrieben.
- Fig. 1 bis Fig. 12:: Auftrag der % Zellpopulationen in der G1-, S- und G2/M-Phase von MCF-7- oder SAOS-2-Zellen und in der An- oder Abwesenheit verschiedener erfindungsgemäßer Peptide
- Fig. 13:: Zytotoxischer Effekt des P3-Peptids auf K562-Zellen
- Fig. 14:: Zytotoxischer Effekt des P3-Peptids auf CCRF-CEM und CCRF-CEM/ACT 400 - Zellen
- Fig. 15:: Effekt des P3-Peptids auf normale periphere mononukleäre Blutzellen
- Tab 1:: Bremsen der Zellzyklusprogression von MCF-7-Zellen durch P5 und P6, nicht aber durch Penetratin
- Tab 2:: Bremsen der Zellzyklusprogression von SAOS-2-Zellen durch P5 und P6, nicht aber durch Penetratin

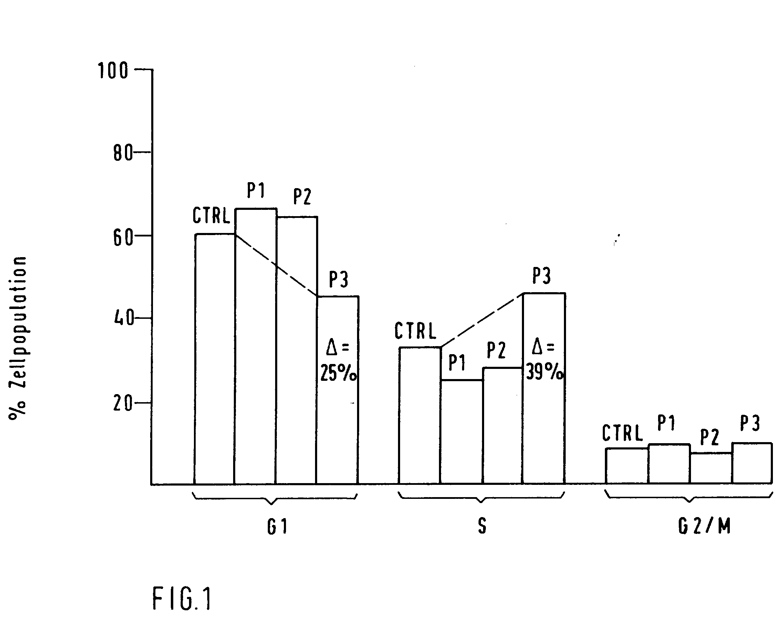

Fig. 1 zeigt, daß das erfindungsgemäße Peptid P3[10⁻⁵M] die G1-Phase vermindert und die S-Phase in der G0/G1-Phase synchronisierter MCF-7-Zellen unter serumfreien Bedingungen erhöht. Die Morphologie der mit P3 behandelten Zellen entspricht der apoptotischer Zellen.

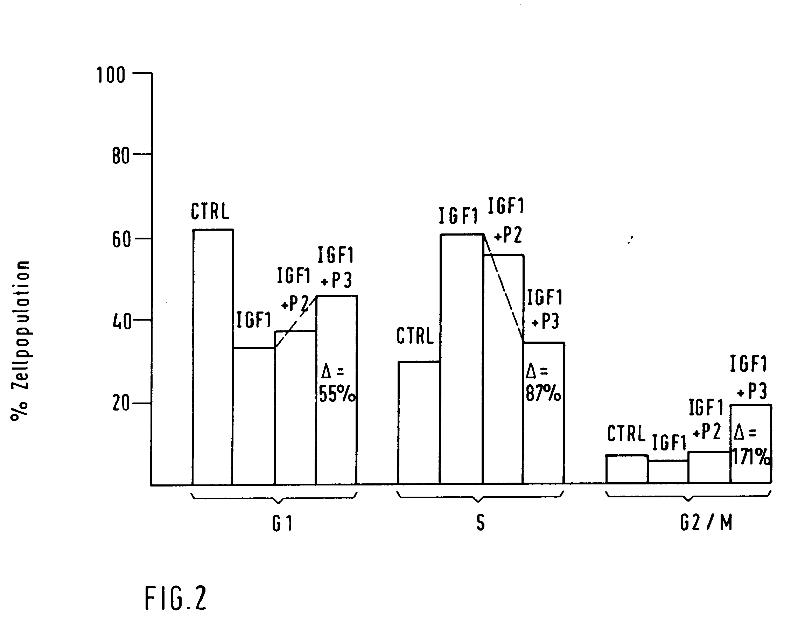

Fig. 2 zeigt, daß das erfindungsgemäße Peptid P3[10⁻ ⁵M] zu einer Zunahme der G1- und G2/M-Phasen sowie zu einer Abnahme der S-Phase von MCF-7-Zellen, die durch insulin-like growth factor 1, kurz IGF-1, in einer optimalen Konzentration von [10⁻⁸M] stimuliert wurden führt. P3 blockiert damit den Effekt von IGF-1 auf MCF-7 Zellen, genauer: das durch IGF-1 bedingte Fortschreiten des Zellzyklus und damit die Zellteilung wird durch P3 gebremst. Die Morphologie der mit P3 behandelten Zellen entspricht der apoptotischer Zellen.

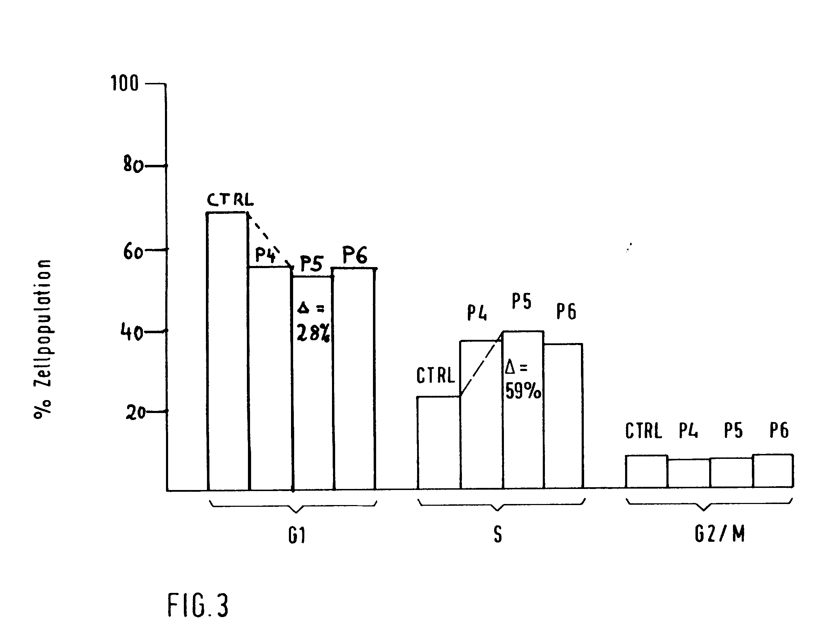

Fig. 3 zeigt, daß jedes der erfindungsgemäßen Peptide P4, P5 und P6 - jeweils in einer Konzentration von 10⁻⁵M - die G1-Phase vermindert und die S-Phase in der G0/G1-Phase synchronisierter MCF-7-Zellen unter serumfreien Bedingungen steigert. Die Morphologie insbesondere der mit P6 behandelten Zellen entspricht der apoptotischer Zellen.

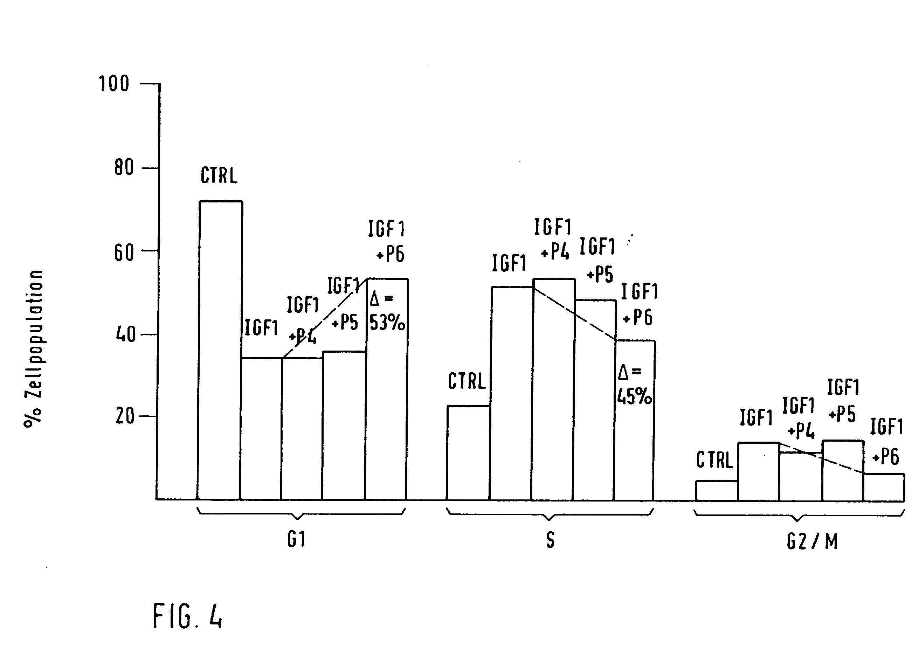

Fig. 4 zeigt, daß das erfindungsgemäße Peptid P6 [10⁻ ⁵M] zu einer Zunahme der G1-Phase sowie einer Abnahme der S-Phase von MCF-7-Zellen führt, die durch IGF-1 in einer optimalen Konzentration von [10⁻⁸M] stimuliert wurden. P6 blockiert damit den Effekt von IGF-1 auf MCF-7 Zellen, genauer: das durch IGF-1 bedingte Fortschreiten des Zellzyklus und damit die Zellteilung wird durch P6 gebremst. Die Morphologie der mit P6 behandelten Zellen entspricht der apoptotischer Zellen.

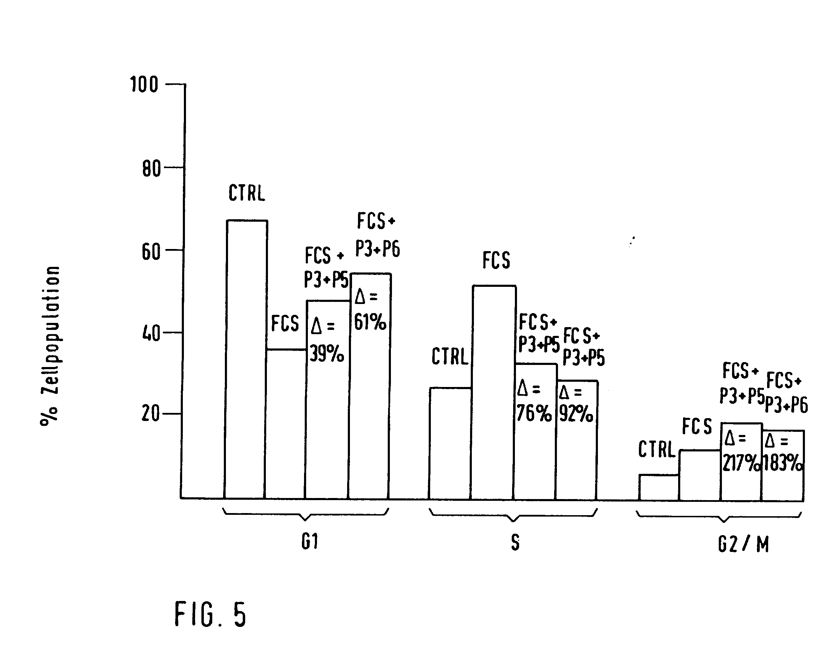

Fig. 5 zeigt, daß die die Kombination der erfindungsgemäßen Peptide P3 [10⁻⁵M] und P5 [10⁻⁵M] sowie die Kombination der erfindungsgemäßen Peptide P3 [10⁻⁵M] und P6 [10⁻⁵M] jeweils zu einer Zunahme der G1 und G2/M-Phasen sowie einer Abnahme der S-Phase von MCF-7-Zellen führt, die durch 10 % Fetales Kalbsserum (kurz: FCS) stimuliert wurden. Die Morphologie der mit diesen Peptidkombinationen behandelten Zellen entspricht jeweils der apoptotischer Zellen.

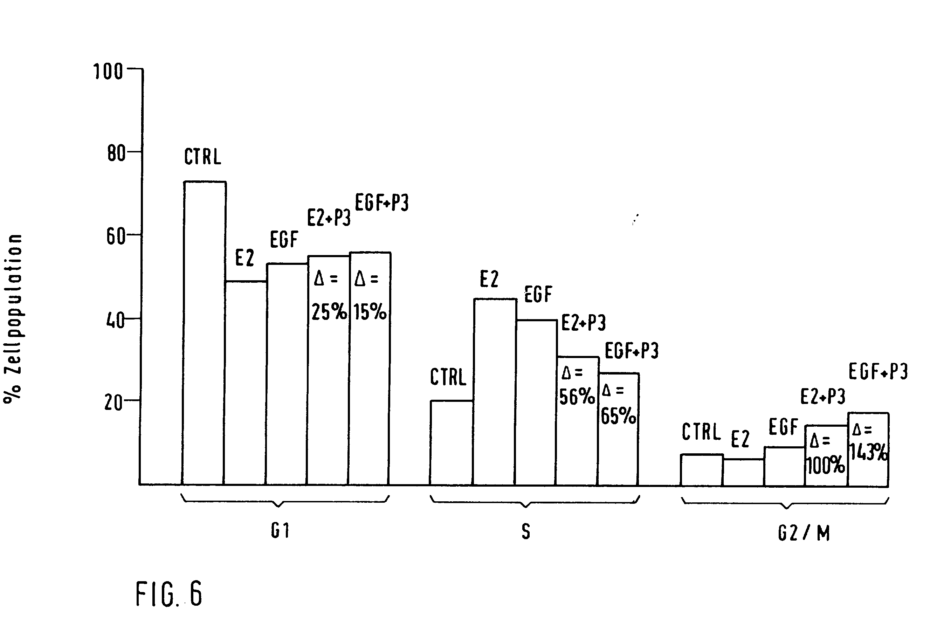

Fig. 6 zeigt, daß das erfindungsgemäße Peptid P3 [10⁻ ⁵M] zu einer Zunahme der G1- und G2-Phasen sowie einer Abnahme der S-Phase von MCF-7-Zellen führt, die durch Östradiol, kurz E2, in einer optimalen Konzentration von [10⁻⁹M] bzw. durch epidermal growth factor, kurz: EGF, in einer optimalen Konzentration von [10⁻⁸] stimuliert wurden. Die Morphologie der mit P3 behandelten Zellen entspricht der apoptotischer Zellen.

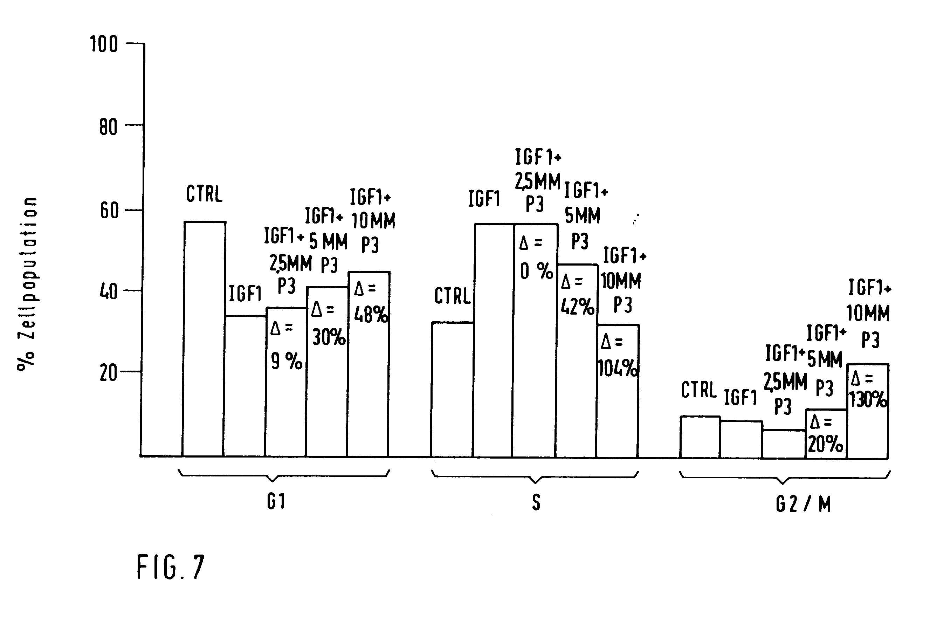

Fig. 7 zeigt, daß das erfindungsgemäße Peptid P3 das durch IGF-1 [10⁻⁸M] bedingte Fortschreiten des Zellzyklus in einer konzentrationsabhängigen Weise blockiert. Die Morphologie der mit P3 [5 x 10⁻⁶M] und insbesondere mit P3 [10⁻⁵M] behandelten Zellen entspricht jeweils der apoptotischer Zellen.

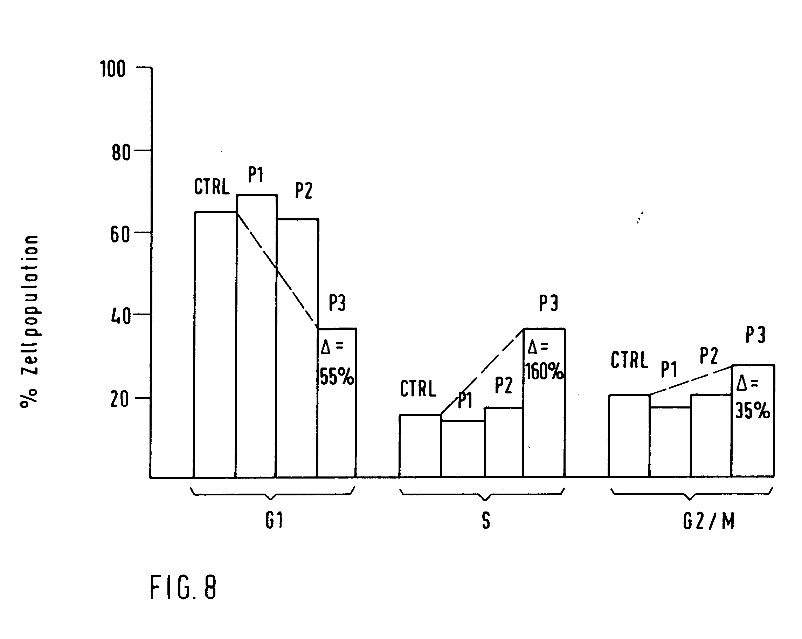

Fig. 8, macht deutlich, daß das erfindungsgemäße Peptid P3 [10⁻⁵M] die G1-Phase vermindert und die S- und G2-Phasen in der G0/G1-Phase synchronisierter SAOS-2-Zellen unter serumfreien Bedingungen erhöht. Die Morphologie der mit P3 behandelten Zellen entspricht der apoptotischer Zellen.

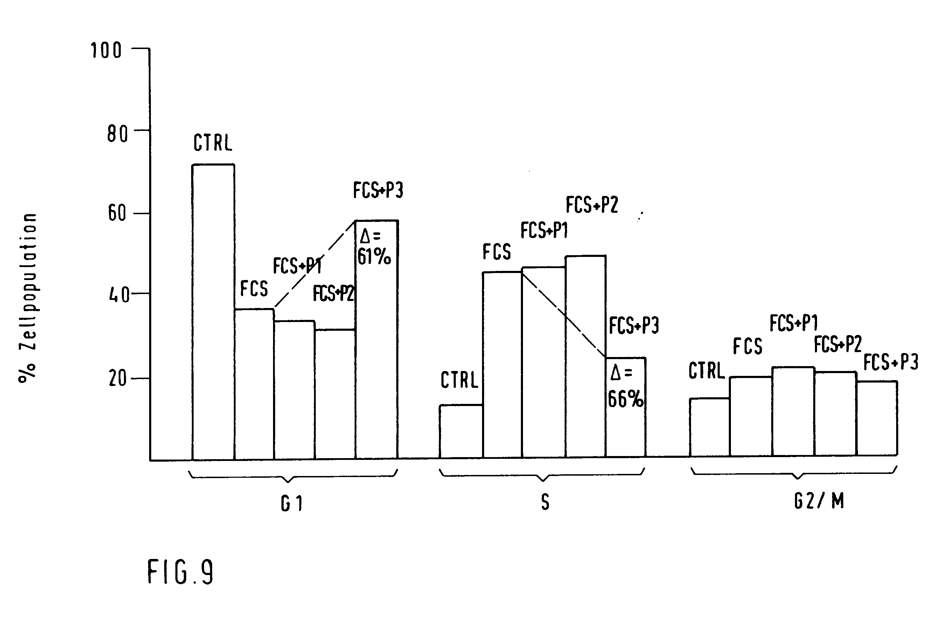

Fig. 9 zeigt, daß das erfindungsgemäße Peptid P3 [10⁻ ⁵M] zu einer Zunahme der G1-Phase sowie einer Abnahme der S-Phase von SAOS-2-Zellen führt, die durch 10 % FS stimuliert wurden. Die Morphologie der mit P3 behandelten Zellen entspricht der apoptotischer Zellen.

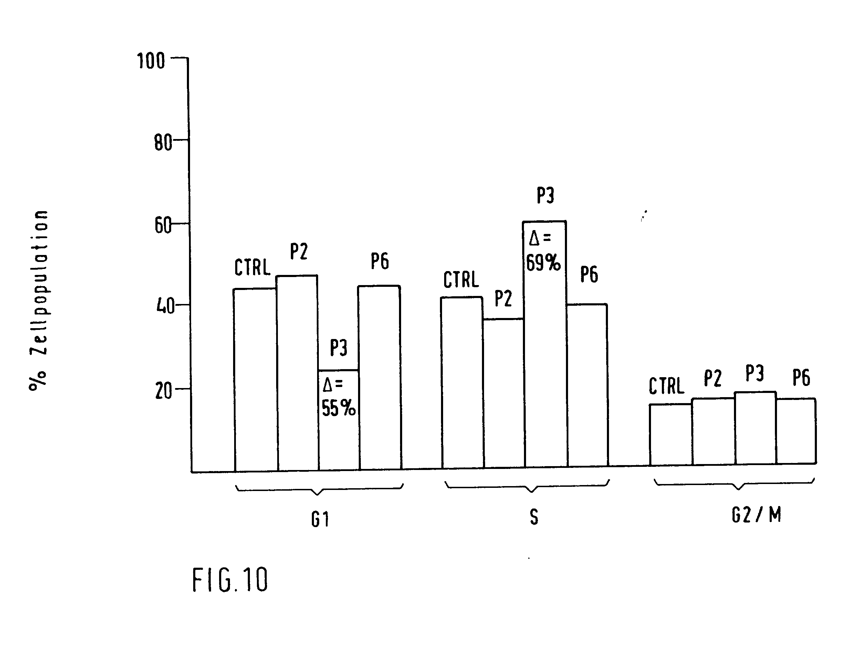

Fig. 10 zeigt, daß das erfindungsgemäße Peptid P3 [10⁻⁵M] die G1-Phase vermindert und die S-Phase von asynchronen MCF-7-Zellen erhöht, die in DMEM-Zellkulturmedium mit 10 % FCS inkubiert wurden. Die Morphologie der mit P3 behandelten Zellen entspricht der apoptotischer Zellen.

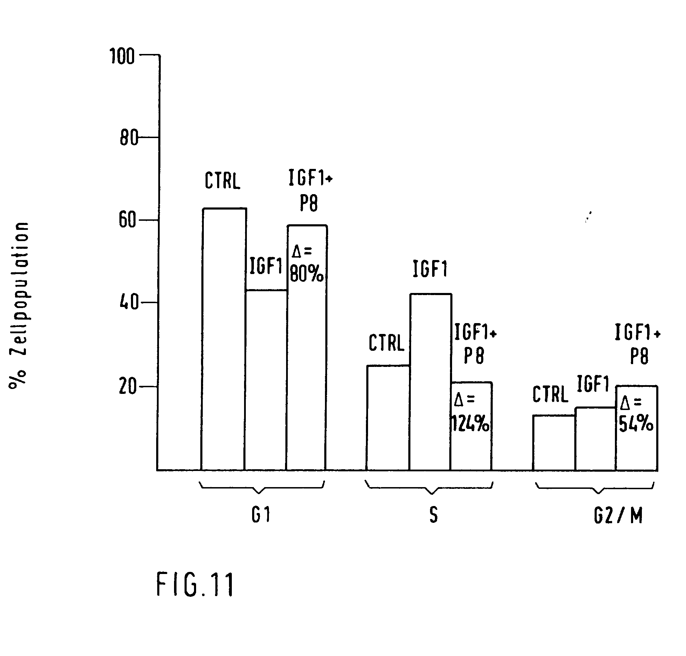

Fig. 11 zeigt, daß das erfindungsgemäße Peptid P8 [10⁻⁵M] zu einer Zunahme der G1- und G2/M-Phasen sowie einer Abnahme der S-Phase von MCF-7-Zellen führt, die durch IGF-1 in einer optimalen Konzentration von [10⁻ ⁸M] stimuliert wurden. P8 blockiert damit den Effekt von IGF-1 auf MCF-7-Zellen, genauer: das durch IGF-1 bedingte Fortschreiten des Zellzyklus und damit die Zellteilung wird durch P8 gebremst. Die Morphologie der mit P8 behandelten Zellen entspricht der apoptotischer Zellen.

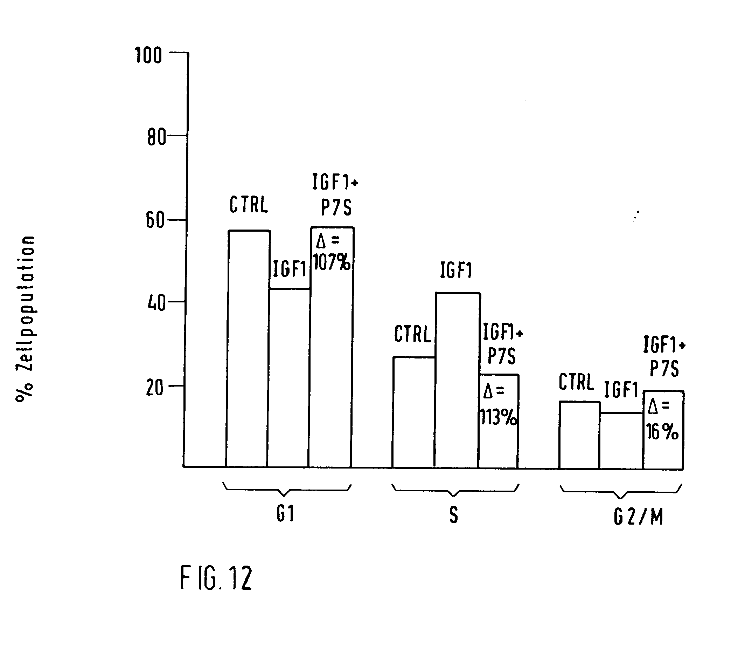

Fig. 12 zeigt, daß das erfindungsgemäße Peptid P7s [10⁻⁵M] zu einer Zunahme der G1- und G2-/M-Phasen sowie einer Abnahme der S-Phase von MCF-7-Zellen führt, die durch IGF-1 in einer optimalen Konzentration von [10⁻⁸M] stimuliert wurden. P7s blockiert damit den Effekt von IGF-1 auf MCF-7-Zellen, genauer: das durch IGF-1 bedingte Fortschreiten des Zellzyklus und damit die Zellteilung wird durch P7s gebremst. Die Morphologie der mit P7s behandelten Zellen entspricht der apoptotischer Zellen.

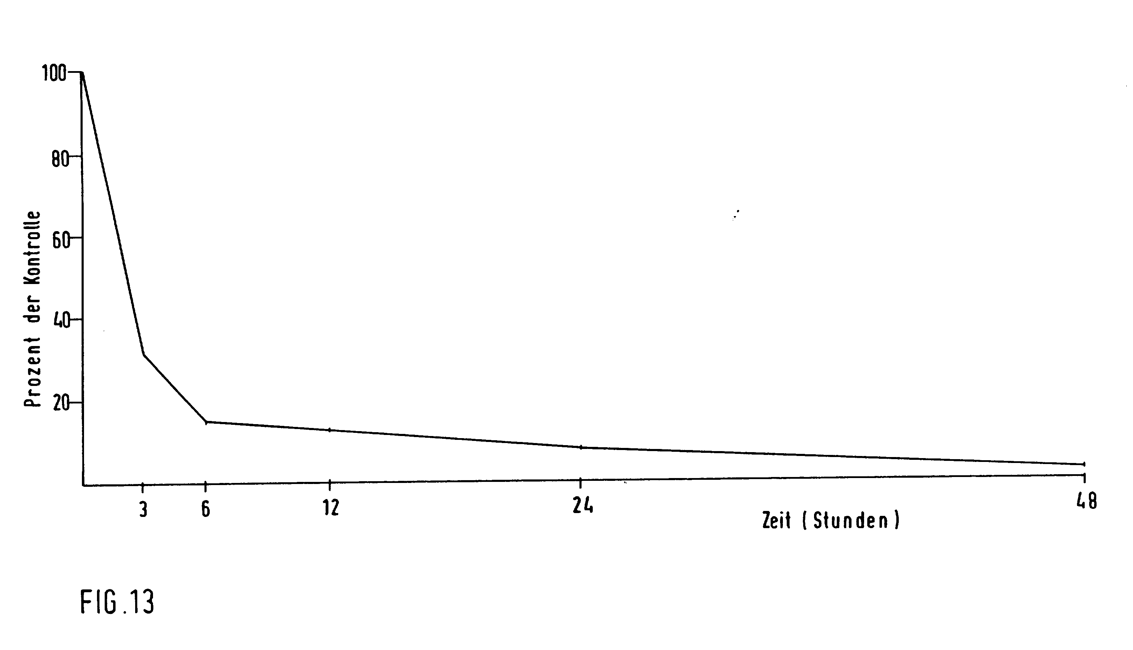

Fig. 13 zeigt, daß der zytotoxische Effekt des erfindungsgemäßen Peptids P3 [10⁻⁵M] auf asynchrone K562-Zellen, die in RPMI-Zellkulturmedium mit 10 % FCS inkubiert wurden, zeitabhängig ist. Die Ergebnisse wurden aufgetragen als lebende mit P3 behandelte Zellen als Prozent der lebenden mit P3 nicht behandelten Zellen. Die Zahl der lebenden Zellen wurde ermittelt, indem man mindestens 200 Zellen mit der Trypanblaumethode zu jedem angegebenen Zeitpunkt auszählte.

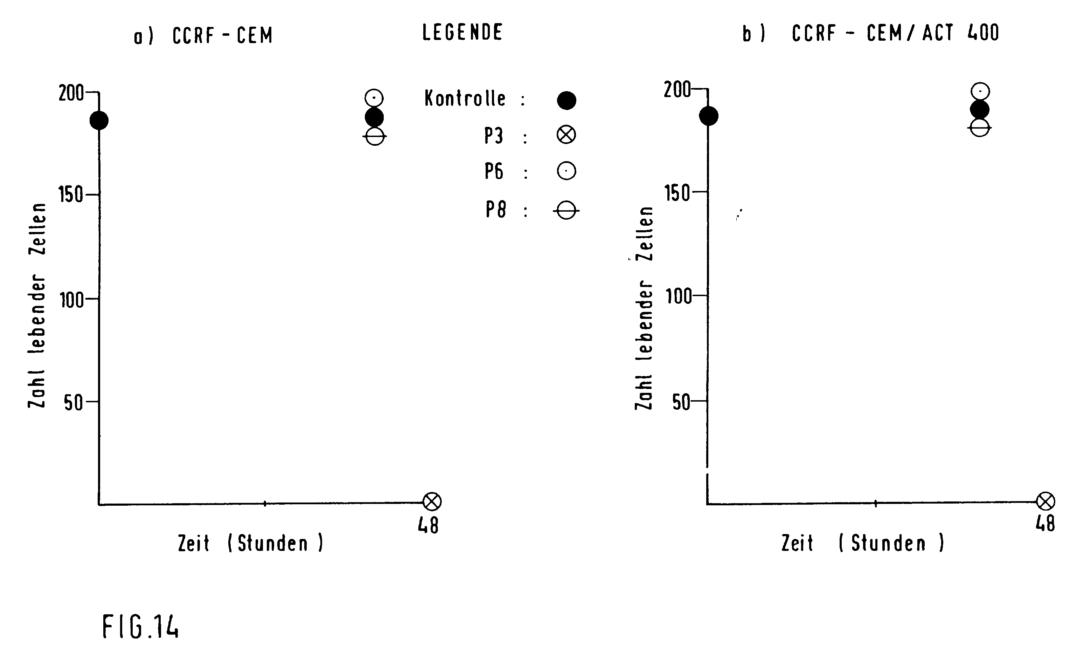

Fig. 14 zeigt, daß das erfindungsgemäße Peptid P3 [10⁻⁵] zytotoxisch auf asynchrone CCRF-CEMₛₑₙₛᵢₜᵢᵥ- und CCRF-CEM/ACT 400ᵣₑₛᵢₛₜₑₙₜ-Zellen wirkt, die in RPMI-Zellkulturmedium mit 10 % FCS inkubiert wurden.

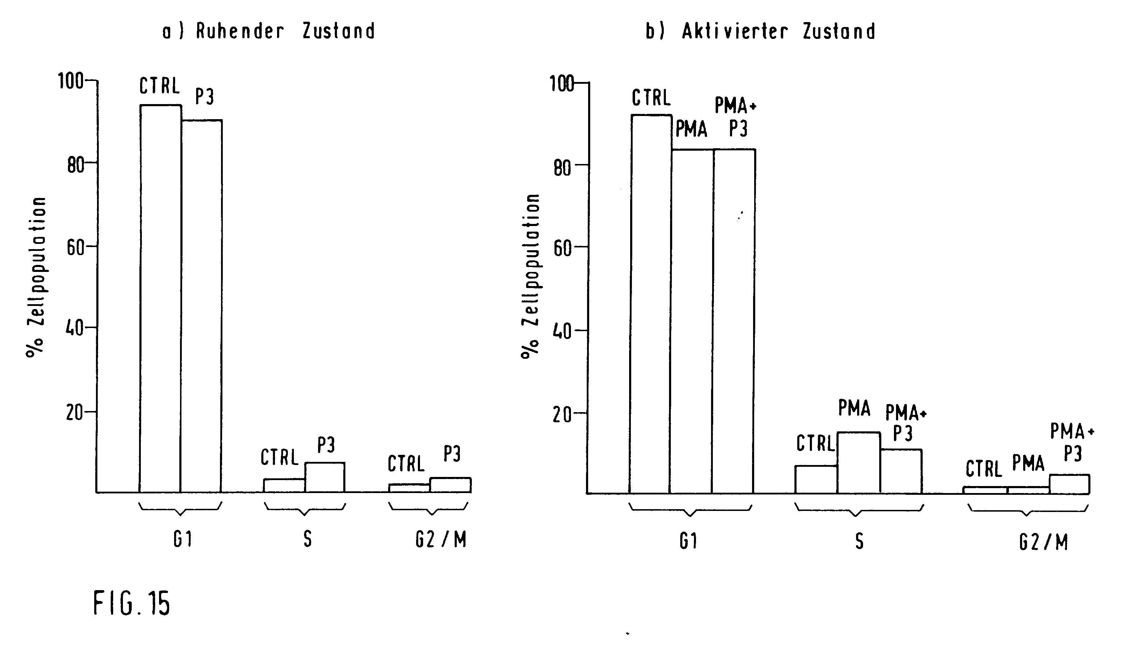

Fig. 15 zeigt, daß das erfindungsgemäße Peptid P3 [10⁻⁵] keinen Effekt auf normale humane mononukleäre Zellen des peripheren Blutes hat, unabhängig davon, ob diese sich in einem ruhenden (a) oder aktivierten (b) Zustand befinden. Entsprechend weisen die mit P3 behandelten Zellen auch keine morphologischen Veränderungen auf. Die Inkubation der Zellen mit P3 erfolgte für einen Zeitraum von 24 Stunden.

Tab. 1 zeigt, daß die erfindungsgemäßen Peptide P5 und P6, je in einer Konzentration von 10⁻⁵M, die S-Phase von MCF-7-Zellen vermindern, die durch IGF-1[10⁻⁸M] bzw. durch Insulin [10⁻⁶M] stimuliert wurden. Das Peptid Penetratin [10⁻⁵M] hat keinen Effekt in diesem Assay, so wie erwartet.

Tab. 2 zeigt, daß die erfindungsgemäßen Peptide P5 und P6, je in einer Konzentration von 5 x 10⁻⁵M, zu einer Zunahme der G1-Phase und einer Abnahme der S-Phase von SAOS-2-Zellen führen, die durch 10 % FCS stimuliert wurden.

Insgesamt läßt sich anhand der beiliegenden Figuren 1-15 sowie der Tabellen 1-2 feststellen, daß die erfindungsgemäßen Peptide P3, P5, P6, P7s oder P8 über die Eigenschaft verfügen, das Fortschreiten des Zellzyklus und damit die Zellteilung der MCF-7-Mammakarzinom (=Brustkrebs)-Zellen, der SAOS-2-Osteosarkom (=Knochenkrebs)-Zellen oder von Leukämie (=Blutkrebs)-Zellen (K562, CCRF-CEM_{Sensitiv}, CCRF-CEM/ACT 400ᵣₑₛᵢ₋ ₛₜₑₙₜ)unter bestimmten Zellkultur-Bedingungen nachhaltig zu bremsen, und zwar allein oder/und in Kombination. Die Daten, die für IGF-1[10⁻⁸M] hier gezeigt werden, gelten auch für Insulin [10⁻⁶M].

Diese Peptide haben damit das Potential, auch in vivo im Menschen als wirksame antineoplastische Substanzen gegen das Krebswachstum zu agieren.

Die Erfindung wird nun weiter durch die Beispiele erläutert:

### Beispiele

Die eine Versuchsanordnung beinhaltet Versuche mit der humanen intaktes Retinoblastomprotein enthaltenden Mammakarzinom-Zell-Linie MCF-7 bzw. der humanen defektes Retinoblastomprotein enthaltenden Osteosarkom-Zell-Linie SAOS-2. Man sät die Zellen zuerst in 12-well-Platten 100.000 (= Hunderttausend) Zellen/-well/ml RPMI oder DMEM/10% FCS) aus, läßt sie 24 Stunden adhärieren, hungert sie anschließend 3 Tage in DMEM ohne FCS mit dem Zweck, die Zellen in der G0/G1-Phase zu synchronisieren, und stimuliert sie dann entweder mit 10% FCS oder 10⁻⁸ M IGF-1 oder 10⁻⁶ M Insulin für 24 Stunden, wobei der Effekt der jeweils zugegebenen Peptide auf die jeweilige Stimulation, die ein Maß für die Zellteilungsrate ist, untersucht wird. Die Kontrollgruppe (G0/G1-synchronisierte Zellen) wird zum Zeitpunkt 0 nach der dreitägigen Hungerphase fixiert, die restlichen Zellen (+/- Peptid) werden nach 24 Stunden fixiert. Daraufhin werden die Zellen zwecks Zellzyklusanalyse im FACS analysiert. Analoge Methoden finden sich beispielsweise bei R. Fahraeus et al., Current Biology, 1996, Vol. 6, No. 1, pp 84-91 sowie bei L. Zhu et al., Genes & Development, 1993, Vol. 7, pp. 1111-1125.

Die zweite Versuchsanordnung betrifft die Leukämie-Zell-Linien K562 und CCRF-CEM und CCRF-CEM/ACT400. Hierbei wurden jeweils 100.000 asynchrone Zellen/well/ml RPMI/10% FCS für 48 Stunden mit den jeweiligen Peptiden in 6-well-Platten inkubiert. Als Readout gilt die Anzahl der mit Hilfe der Trypanblaufärbung (L.D. Attardi et al., The EMBO Journal, 1996, Vol. 15, No. 14, pp. 3693-3701 und M.K. Reeder & H.C. Isom, Cell Growth & Differentiation, 1996, Vol. 7, pp. 449-460) nach 48 Stunden Inkubationszeit festgestellten toten Zellen/200 ausgezählte Zellen. Je mehr tote Zellen, desto zytotoxischer das Peptid und damit umso effektiver.

Die Ergebnisse der Beispiele sind in den Figuren 1 bis 15 sowie in den Tabellen 1 und 2 gezeigt.

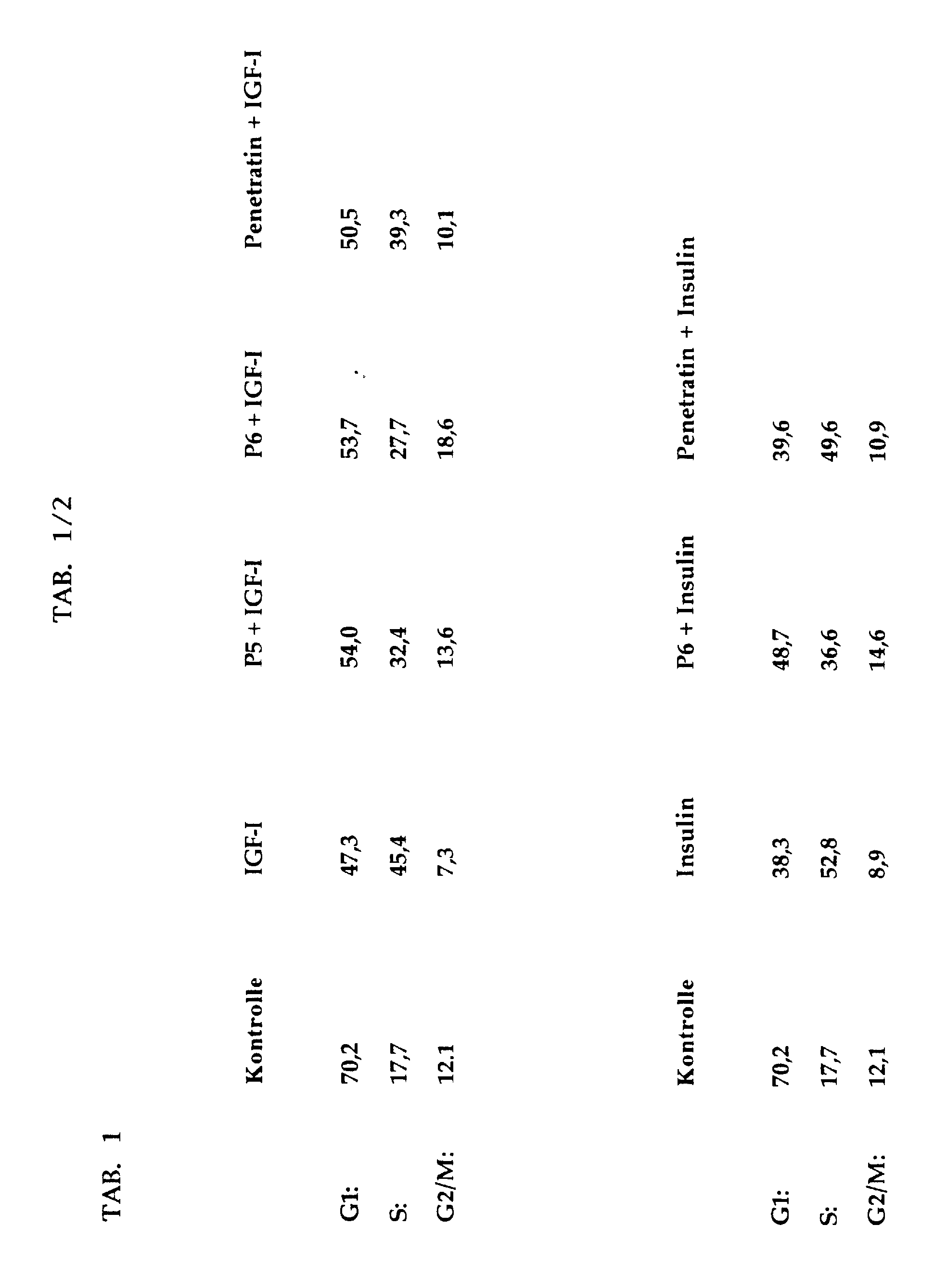

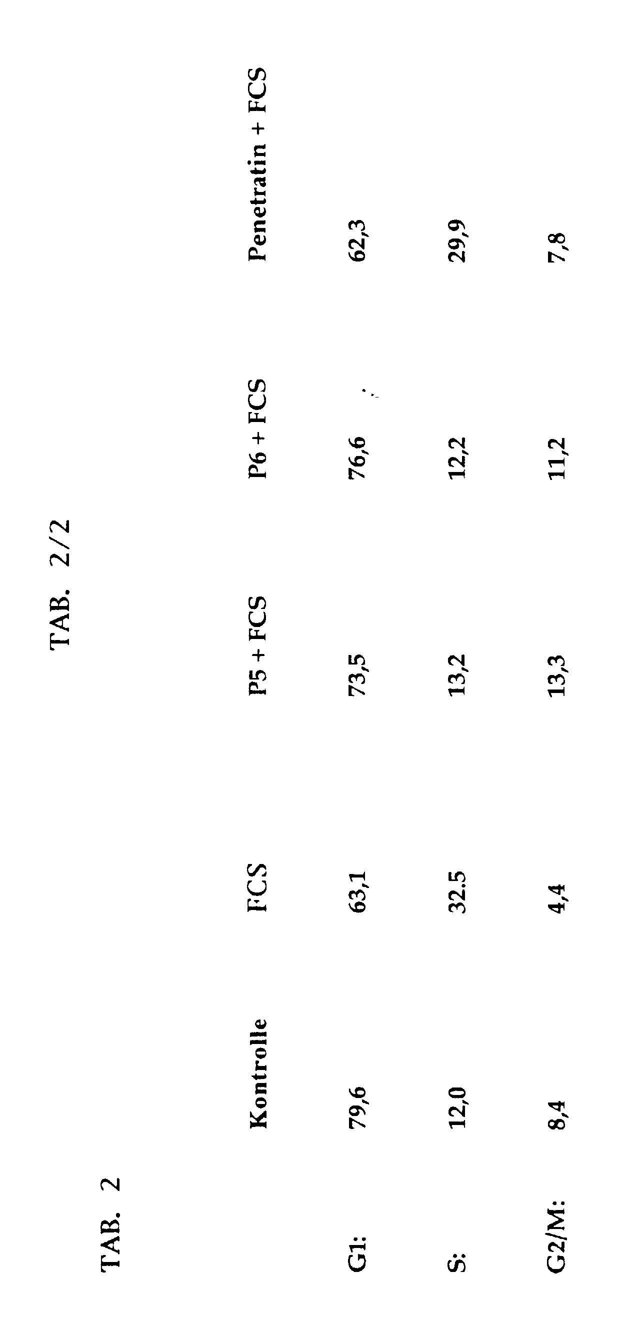

## Patentansprüche

1. Antiproliferatives Peptid AB oder BA, bestehend aus:
- einem antiproliferativen Teil (A), umfassend ein Fragment eines Tumorsuppressorproteins oder ein dazu hydropathisch homologes Peptidsegment, das an ein Wachstumsfaktor- oder Wachstumsfaktorrezeptor-S bindet,
- einem Teil (B), der als Kofaktor den Teil (A) gegen Proteasen stabilisiert.

2. Peptid nach Anspruch 1,
wobei der antiproliferative Teil (A) zu einem Wachstumsfaktor- oder einem Wachstumsfaktorrezeptor-Segment hydropathisch komplementär ist.

3. Peptid nach Anspruch 1 oder 2,
wobei der antiproliferative Teil (A) ein von dem Retinoblastomprotein (RB1) abgeleitetes Tumorsuppressorprotein-Fragment ist.

4. Peptid nach einem der Ansprüche 1 bis 3,
wobei der Teil (A) die Aminosäuresequenz FYKK oder eine in mindestens zwei Positionen dazu hydropathisch homologe Aminosäuresequenz umfaßt.

5. Peptid nach Anspruch 4,
wobei der Teil (A) die Aminosäuresequenz LFYKKV oder eine in mindestens zwei Positionen dazu hydropathisch homologe Aminosäuresequenz umfaßt.

6. Peptid nach einem der vorhergehenden Ansprüche,
wobei der Teil (B) ausgewählt ist unter einem verzweigten Peptid, einem Polylysin-Core, D-Aminosäuren und einer nukleären Lokalisationssequenz (NLS).

7. Peptid nach Anspruch 6,
wobei der Teil (B) ein Polylysin-Core ist, der ausgewählt ist unter
a) [GGG]₄[KRG]₂KG
b) [GGG]₄[K]₂KG
c) [GGG]₄[IKdRG]₂lKG.

8. Peptid nach Anspruch 6.
wobei der Teil (B) eine NLS ist, die ausgewählt ist unter
a) PKKKRKV
b) RQIKIWFQNRRMKWKK
c) einer bipartiten NLS
d) der RNP A1 NLS.

9. Peptid nach einem der Ansprüche 1 bis 8, wobei das Wachstumsfaktor-Segment oder das Wachstumsfaktorrezeptor-Segment eine Aminosäuresequenz mit dem folgenden hydropathischen Profil umfaßt:
hydrophobe Aminosäure - X - hydrophobe Aminosäure - X - hydrophile Aminosäure, worin X eine beliebige Aminosäure darstellt.

10. Peptid nach Anspruch 9,
wobei das Wachstumsfaktor-Segment oder das Wachstumsfaktorrezeptor-Segment die Aminosäuresequenz LXCXE oder FVCGD umfaßt,
wobei X eine beliebige Aminosäure darstellt.

11. Peptid nach Anspruch 10,
wobei die Aminosäuresequenz LXCXE von Insulin abgeleitet ist.

12. Peptid nach einem der Ansprüche 1 bis 11,
wobei das Peptid ausgewählt ist unter
a) [LFYKKVGGG]₄[KRG]₂KG
b) [dLdFdYdKdKdVGGG]₄[IKdRG]₂1KG
c) [dLdFdYdKdKdVGGG]₄[lK]₂1KG
d) LFYKKVPKKKRKV
e) (all-D) LFYKKVPKKKRKV
f) LFYKKVRQIKIWFQNRRMKWKK
g) (all-D) LFYKKVRQIKIWFQNRRMKWKK
h) [dKdVdLdYdFdKGGG]₄[IKdRG]₂lKG
i) [dKdVdLdYdFdKGGG]₄[1K]₂lKG
j) KVLYFKRQIKIWFQNRRMKWKK
k) (all-D) KVLYFKRQIKIWFQNRRMKWKK.

13. Nukleinsäure kodierend für ein Peptid nach einem der vorhergehenden Ansprüche, entsprechend dem genetischen Code.

14. Nukleinsäure nach Anspruch 13,
wobei die Nukleinsäure die folgende Sequenz umfaßt:
a) 5'- UUC UAC AAG AAG - 3'     (D0) oder eine für das gleiche Peptid kodierende Nukleinsäure,
b) in der ein oder mehrere Nukleotide ersetzt sind, oder
c) die mit der Nukleinsäure (D0) hybridisiert, oder
d) die mit der Nukleinsäure (D0) über den degenerierten genetischen Code verwandt ist.

15. Nukleinsäure nach Anspruch 13,
wobei die Nukleinsäure die folgende Sequenz umfaßt:
a) 5'- CUU UUC UAC AAG AAG GUU - 3' (D1) oder eine für das gleiche Peptid kodierende Nukleinsäure, in der
b) ein oder mehrere Nukleotide ersetzt sind, oder
c) die mit der Nukleinsäure (D1) hybridisiert, oder
d) die mit der Nukleinsäure (D1) über den degenerierten genetischen Code verwandt ist.

16. Peptidnukleinsäure, deren Struktur homolog ist zu der einer Nukleinsäure nach einem der Ansprüche 13 bis 15.

17. Pharmazeutische Zusammensetzung, welche ein oder mehrere Peptide nach einem der Ansprüche 1 bis 12 enthält.

18. Pharmazeutische Zusammensetzung nach Anspruch 17 in Form von Salben, Lösungen, Dispersionen, Emulsionen, Aerosolen, Schäumen, teilchenförmigen Mitteln, Pillen, Pastillen, Tabletten, Dragees oder Kapseln.

19. Pharmazeutische Zusammensetzung nach Anspruch 17,
bei der die teilchenförmigen Mittel ausgewählt werden unter Granulaten, Agglomeraten, Puder, Mikroperlen und Adsorbaten.

20. Verwendung eines Peptids nach einem der Ansprüche 1 bis 12 zur Herstellung eines Arzneimittels zur Diagnose und/oder Therapie von Erkrankungen, die mit einer gesteigerten Proliferation bzw. Hyperproliferation von Zellen einhergehen.

21. Verwendung einer Nukleinsäure nach einem der Ansprüche 13 bis 15 zur Herstellung eines Arzneimittels zur Diagnose und/oder Therapie von Erkrankungen, die mit einer gesteigerten Proliferation bzw. Hyperproliferation von Zellen einhergehen.

22. Verwendung einer Peptidnukleinsäure nach Anspruch 16 zur Herstellung eines Arzneimittels zur Diagnose und/oder Therapie von Erkrankungen, die mit einer gesteigerten Proliferation bzw. Hyperproliferation von Zellen einhergehen.

23. Verwendung einer pharmazeutischen Zusammensetzung nach einem der Ansprüche 17 bis 19 zur Herstellung eines Arzneimittels zur Diagnose und/oder Therapie von Erkrankungen, die mit einer gesteigerten Proliferation bzw. Hyperproliferation von Zellen einhergehen.

24. Verwendung nach einem der Ansprüche 20 bis 23, wobei die Erkrankung ein benigner Tumor, ein maligner Tumor oder Atherosklerose ist.

25. Verfahren zur Reingewinnung von Wachstumsfaktoren und/oder Wachstumsfaktor-rezeptoren, wobei ein Peptid nach einem der Ansprüche 1 bis 12 an einen festen Träger gekoppelt wird, mit der Maßgabe, daß das Peptid [LFYKKVGGG]₄[K]₂KG ausgeschlossen ist.

26. Verfahren zur Identifikation oder Klonierung von Wachstumsfaktoren, Wachstumsfaktorrezeptoren und/oder Tumorsuppressorproteinen, wobei die Sequenz eines Peptids nach einem der Ansprüche 1 bis 12, die Sequenz einer Nukleinsäure nach einem der Ansprüche 13 bis 15 und/oder die Sequenz einer Peptidnukleinsäure nach Anspruch 16 verwendet wird/werden.

27. Verfahren zur Vorhersage von Komplexbildungen zwischen Wachstumsfaktoren/ Wachstumsfaktorrezeptoren und Tumorsuppressorproteinen, wobei die cDNA, die für einen Wachstumsfaktor, einen Wachstumsfaktorrezeptor oder ein Tumorsuppressorprotein kodiert, in eine komplementäre DNA-Sequenz übersetzt wird, die in eine Peptidsequenz übersetzt wird, welche dazu verwendet wird, mit dieser Sequenz identische oder homologe Segmente von Proteinen oder Peptiden in Datenbanken zu finden.
